Europäisches Patentamt

European Patent Office

Office européen des brevets

(11) Veröffentlichungsnummer : **0 169 974
B1**

(12) **EUROPÄISCHE PATENTSCHRIFT**

(45) Veröffentlichungstag der Patentschrift :
07.09.88

(21) Anmeldenummer : 85104235.8

(22) Anmeldetag : 06.04.85

(51) Int. Cl.⁴ : **A 61 F   2/34**

(54) **Randlose Pfannenprothese für ein Hüftgelenk.**

(30) Priorität : 03.07.84 CH 3182/84

(43) Veröffentlichungstag der Anmeldung :
05.02.86 Patentblatt 86/06

(45) Bekanntmachung des Hinweises auf die Patenterteilung : 07.09.88 Patentblatt 88/36

(84) Benannte Vertragsstaaten :
AT DE FR GB IT

(56) Entgegenhaltungen :
DE-A- 3 228 113
FR-A- 2 197 561
FR-A- 2 225 924
US-A- 3 840 904
US-A- 3 903 549

(73) Patentinhaber : GEBRÜDER SULZER AKTIENGESELL-
SCHAFT
Zürcherstrasse 9
CH-8401 Winterthur (CH)

(72) Erfinder : Griss, Peter, Prof. Dr.-Med.
Klinikum Mannheim
D-6800 Mannheim (DE)

(74) Vertreter : Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)

EP 0 169 974 B1

Jouve, 18, rue St-Denis, 75001 Paris, France

**Beschreibung**

Die Erfindung betrifft eine Pfannenprothese für ein Hüftgelenk, deren Pfannenkörper halbkugelförmig ausgebildet und im Bereich der Äquatorebene mit drei aus seiner Kegelfläche hervorstehenden, sich im wesentlichen in Meridianrichtung erstreckenden Flügeln versehen ist, die auf dem Umfang so verteilt sind, daß ein mittlerer Flügel in einem ersten Winkel $\alpha$ zu einem auf einer Seite benachbarten Flügel und in einem zweiten Winkel $\beta$ zu dem auf der anderen Seite benachbarten Flügel angeordnet ist, wobei für den Winkel $\beta$ $85° \leq \beta \leq 105°$ gilt. Eine derartige Prothese ist aus der US-A-3 903 549 bekannt, die mit einem äquatorialen Rand versehen ist und drei Flügel zur Verstärkung der Halbkugelschale und zur Fixierung des Randes besitzt. Die Flügel schließen untereinander Winkel $\alpha$ und $\beta$ von jeweils 90° ein. Für eine zementfreie Fixierung im Beckenknochen sind Knochenschrauben vorgesehen, die durch den äquatorialen Rand geführt sind.

Eine randlose Hüftgelenkendoprothese ist aus der DE-A-3 228 113 bekannt ; bei ihr sind zwei Keile im Äquatorbereich als Rotationssicherung vorgesehen. Die Keile schließen untereinander und mit einem in eine Knochenbohrung eingreifenden Stützzapfen je einen Winkel von 120° ein.

Zur zementfreien Verankerung im Becken werden derartige Pfannenprothesen entweder in den Knochen eingeschraubt oder eingeschlagen. Während das Einschrauben für den Operateur mühevoll, zeitraubend und schwierig ist, ist neben der Primär auch die Dauer-(Sekundär-) Verankerung und Rotationssicherung bei zementfrei in den Knochen eingeschlagenen Pfannenprothesen häufig ungenügend, da der Knochen besonders durch aus der Pfannenoberfläche hervorstehende Verankerungselemente, wie Flügel, Zapfen, Rippen oder Warzen, örtlich überlastet wird. Derartige Überlastungen können zu Knochenfisuren und -abbau führen, aus denen oftmals im Laufe der Zeit Lockerungen resultieren.

Aufgabe der Erfindung ist es daher, eine in das Becken zementfrei einzuschlagende Pfannenprothese zu schaffen, die einen festen Sitz und eine ausreichende Rotationssicherung über einen langen Zeitraum gewährleistet, ohne daß der Knochen dadurch örtlich überlastet wird. Diese Aufgabe wird erfindungsgemäß dadurch gelöst, daß die Pfannenprothese randlos ist und für den ersten Winkel $135° \leq \alpha \leq 155°$ gilt.

Die neue Pfannenprothese, für die je eine gesonderte Ausführung für die rechte und für die linke Hüfte vorhanden ist, wird so in das Becken eingeschlagen, daß die drei Flügel in ausgesuchte Bereiche des Darm-, Scham- bzw. Sitzbeins eindringen, die gegenüber benachbarten Bereichen ein — in Eindringrichtung — « tiefes » und festes Knochengewebe aufweisen. Dabei hat es sich als vorteilhaft erwiesen, wenn der Winkel $\alpha$ zwischen dem Flügel für das Darmbein und demjenigen für das Sitzbein 145° beträgt, während der Darmbeinflügel und der Schambeinflügel einen Winkel $\beta$ von

95° einschließen.

Wird die neue Pfanne darüberhinaus in bekannter Weise mit einem im wesentlichen in Richtung der Hauptbelastung verlaufenden Zapfen ausgerüstet, so ist es zweckmässig, wenn die Flügel gegen die Meridianebene durch ihre Schwerpunkte geneigt sind und mindestens annähernd parallel zur Zapfenachse verlaufen. Gleichzeitig können auch die peripheren Aussenkanten der Flügel sich parallel zur Zapfenachse erstrecken. Flügelflächen und periphere Aussenkanten weisen durch diese Neigungen in die Richtung, in der die Prothese in die Knochen eingetrieben wird. Ihr Eindringen in die Knochen wird dadurch erleichtert und darüberhinaus eine gleichmässige Verdrängung von Knochenmaterial bei diesem Eindringen bewirkt. Das Eindringen der Flügel in das nicht oder nur geringfügig operativ ausgenommene Becken — es wird beim Implantieren der neuen Pfannenprothese sehr oft, wenn überhaupt, nur Knorpelgewebe entfernt — kann weiter erleichtert werden, wenn die Flügel mit mindestens im wesentlichen äquatorparallelen Kanten in die Halbkugelfläche einmünden und als messerartige Schneiden ausgebildet sind.

Die neue Pfannenprothese kann aus allen für Pfannenprothesen gebräuchlichen Implantatwerkstoffen gefertigt sein. Vorzugsweise besteht sie jedoch aus Metall, z. B. Titan oder einer Titanlegierung.

Um die Sekundär-Verankerung zu verbessern, ist es selbstverständlich möglich, die Aussenfläche des Pfannenkörpers und/oder den Zapfen in bekannter Weise mit Verankerungsstrukturen, wie Rillen, Noppen, Rippen usw., zu versehen.

Im folgenden wird die Erfindung anhand eines Ausführungsbeispiels im Zusammenhang mit der Zeichnung näher erläutert.

Fig. 1 ist in Richtung des Schenkelhalses des Femurs bzw. der Pfannenachse eine Aufsicht auf die natürliche rechte Hüftgelenkspfanne im Becken ;

Fig. 2 gibt eine Aufsicht auf die Halbkugel der neuen Pfannenprothese wieder ;

Fig. 3 und 4 sind Ansichten von Fig. 2 in Richtung der Pfeile A bzw. B.

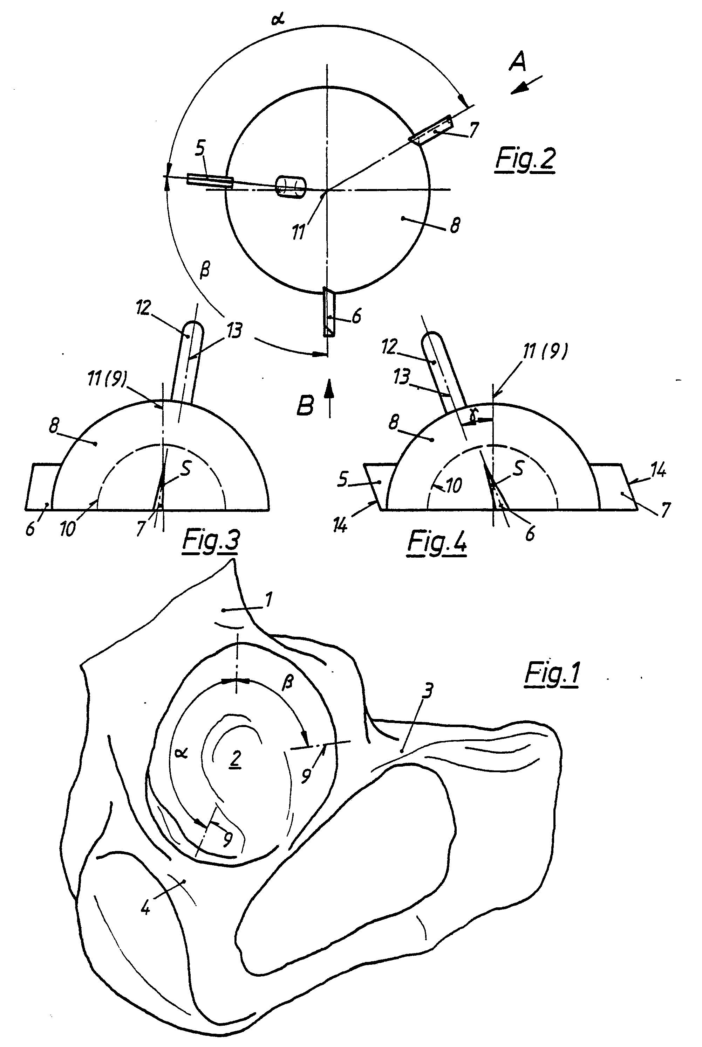

In Fig. 1 begrenzt das nur teilweise dargestellte Darmbein 1 die natürliche Pfannenschale 2 im « nordwestlichen » Bereich ; den rechten Halbbogen der Pfannenschale 2 bildet das Schambein 3, während die zwischen beiden verbleibende Schalenbegrenzung vom Sitzbein 4 gebildet wird. In die Pfannenschale 2 ist schematisch die Lage eingezeichnet, die die Flügel 5 bis 7 (Fig. 2) der eingesetzten Pfannenprothese in den genannten Knochen 1, 3 und 4 etwa einnehmen. Die strichpunktierte Linie repräsentiert dabei die Meridianebene 9 des Pfannenkörpers 8 (Fig. 2), in der der jeweilige Flügelschwerpunkt S liegt, wobei die Winkel in der räumlichen Skizze der Fig. 1 sich nicht mit der ebenen Darstellung der Fig. 2 decken.

Der Darmbeinflügel 5 bildet mit dem Sitzbeinflügel 7 in dem gezeigten Beispiel einen Winkel α von 145°, während sein Winkel β zum Schambeinflügel 6 95° beträgt. An den in Fig. 1 bezeichneten Stellen sind, was in der flächigen Darstellung nicht zu erkennen ist, die die Flügel 5 bis 7 aufnehmenden Knochen besonders fest, kompakt und räumlich « tief ». Das Eindringen der Flügel 5 bis 7 bewirkt daher an diesen Stellen eine intensive Verdrängung und Verdichtung des Gewebes, wodurch sich eine hohe Primärstabilität für die Pfannenprothese und gute Anwachsmöglichkeiten des Gewebes an den Pfannenkörper und die Flügel ergeben, was eine sichere Sekundär-Verankerung gewährleistet.

Der Pfannenkörper 8 bildet, wie aus den Fig. 2 bis 4 zu entnehmen ist, eine randlose Halbkugel, aus der vom Aequator her die künstliche Pfannenschale 10 herausgearbeitet ist. Pfannenkörper 8 und Pfannenschale 10 haben eine gemeinsame Rotationssymmetrieachse 11, die in den Fig. 3 und 4 mit den Meridianebenen 9 zusammenfällt.

In Richtung der Hauptbelastungen ragt aus dem Pfannenkörper 8 ein Verankerungszapfen 12 heraus, dessen Achse 13 mit einer die Achse 11 enthaltenden Mittelebene einen spitzen Winkel γ (Fig. 4) einschliesst. Wie die Fig. 3 und 4 zeigen, sind die Flügel 5 bis 7 gegenüber den Meridianebenen 9 durch ihre Schwerpunkte S derart geneigt, dass ihre Flächen mindestens annähernd parallel zur Zapfenachse 13 verlaufen. Zusätzlich sind die peripheren Aussenkanten 14 der Flügel 5 bis 7, die im Querschnitt messerartige Schneiden bilden, in ihrem Verlauf ebenfalls an die Richtung der Zapfenachse 13 angepasst. Die Ausrichtung der Flügelflächen und der Aussenkanten 14 auf die Zapfenachse 13, die gleichzeitig die Einschlagrichtung der Pfannenprothese bei der Implantation ist, erleichtert die Arbeit des Operateurs und fördert eine gute Haftung der in den Knochen eindringenden Flügel-Schneiden.

## Patentansprüche

1. Pfannenprothese für ein Hüftgelenk, deren Pfannenkörper (8) halbkugelförmig ausgebildet und im Bereich der Äquatorebene mit drei aus seiner Kegelfläche hervorstehenden, sich im wesentlichen in Meridianrichtung erstreckenden Flügeln (5 bis 7) versehen ist, die auf dem Umfang so verteilt sind, daß ein mittlerer Flügel (5) in einem ersten Winkel (α) zu einem auf einer Seite benachbarten Flügel (7) und in einem zweiten Winkel (β) zu dem auf der anderen Seite benachbarten Flügel (6) angeordnet ist, wobei für den Winkel (β) 85°≤β≤105° gilt, dadurch gekennzeichnet, daß die Pfannenprothese randlos ist und für den ersten Winkel (α) 135°≤α≤155° gilt.

2. Pfannenprothese nach Anspruch 1, dadurch gekennzeichnet, dass der erste Winkel (α) 145° und der zweite Winkel (β) 95° betragen.

3. Pfannenprothese nach Anspruch 1, dadurch gekennzeichnet, dass die Fläche mindestens eines der Flügel (5-7) gegen eine Meridianebene (9) durch seinen Schwerpunkt (S) geneigt ist.

4. Pfannenkörper nach Anspruch 3, dadurch gekennzeichnet dass die Prothese mit einem Verankerungszapfen auf ihrer Aussenoberfläche versehen ist, der mit einer die Rotationssymmetrieachse des Pfannenkörpers enthaltenden Mittelebene einen spitzen Winkel einschliesst, wobei die geneigte Flügelfläche mindestens annähernd parallel zur Zapfenachse (13) verläuft.

5. Pfannenkörper nach Anspruch 4, dadurch gekennzeichnet, dass die peripheren Aussenkanten (14) der Flügel (5-7) mindestens im wesentlichen parallel zur Zapfenachse (13) gerichtet sind.

6. Pfannenprothese nach einem der vorhergehenden Ansprüche, dadurch gekennzeichnet, dass die Flügel (5-7) mit mindestens im wesentlichen äquatorparallelen Kanten in die Halbkugelfläche einmünden und als messerartige Schneiden ausgebildet sind.

## Claims

1. An acetabular cup prosthesis whose cup (8) is hemispherical and has near the mid-circumferential plane three webs (5-7), the same projecting from the conical surface of the cup, extending substantially in the meridian direction and being so distributed around the periphery that a central web (5) is disposed at a first angle (α) to an adjacent web (7) disposed on one side and at a second angle (β) to the adjacent web (6) on the other side, for the angle (β) 85°≤β≤105°, characterised in that the cup prosthesis is rimless and for the first angle (α) 135°≤α≤155°.

2. A prosthesis according to claim 1, characterised in that the first angle (α) is 145° and the second angle (β) is 95°.

3. A prosthesis according to claim 3, characterised in that the surface of at least one of the webs (5-7) is inclined towards a meridian plane (9) through its centrel (S) of gravity.

4. A prosthesis according to claim 3, characterised in that it has a fixing nail on its outside surface, the nail including an acute angle with a centre-plane which contains the axis of rotational symmetry of the cup prosthesis, the inclined web surface extending at least substantially parallel to the nail axis (13).

5. A prosthesis according to claim 4, characterised in that the peripheral outside edges (14) of the webs (5-7) are oriented at least substantially parallel to the nail axis (13).

6. A prosthesis according to any of the previous claims, characterised in that the webs (5-7) join the hemispherical surface by way of edges at least substantially parallel to the midcircumferential plane and are in the form of knife-like cutting edges.

## Revendications

1. Prothèse en cuvette pour articulation coxofé- morale, dont le corps de cuvette (8) est de réalisation hémisphérique et est pourvu, au voisinage du plan équatorial, de trois ailettes (5 à 7) qui dépassent de sa surface tronconique, s'étendent pour l'essentiel dans la direction méridienne et sont réparties, sur le pourtour, de telle sorte qu'une ailette centrale (5) soit disposée selon un premier angle (α) par rapport à une ailette (7) voisine d'un côté et selon un second angle (β) par rapport à l'ailette (6) voisine de l'autre côté, l'angle (β) obéissant à la relation 85°≤β≤105°, caractérisée par le fait que cette prothèse en cuvette est dépourvue de collerette, et le premier angle (α) obéit à la relation 135°≤α≤155°.

2. Prothèse en cuvette selon la revendication 1, caractérisée par le fait que le premier angle (α) mesure 145° et le second angle (β) mesure 95°.

3. Prothèse en cuvette selon la revendication 1, caractérisée par le fait que la surface d'au moins l'une des ailettes (5-7) est inclinée vers un plan méridien (9), par son centre de gravité (S).

4. Corps de cuvette selon la revendication 3, caractérisé par le fait que la prothèse est munie, sur sa surface externe, d'un tenon d'ancrage qui forme un angle aigu avec un plan médian renfermant l'axe de symétrie de rotation du corps de cuvette, la surface d'ailette inclinée s'étendant au moins approximativement parallèlement à l'axe (13) du tenon.

5. Corps de cuvette selon la revendication 4, caractérisé par le fait que les arêtes externes périphériques (14) des ailettes (5. 7) sont orientées au moins pour l'essentiel parallèlement à l'axe (13) du tenon.

6. Prothèse en cuvette selon l'une des revendications précédentes, caractérisée par le fait que les ailettes (5. 7) débouchent dans la surface hémisphérique par des arêtes au moins pour l'essentiel parallèles à l'équateur, et sont réalisées en tant que tranchants du type couteaux.

0 169 974

*Fig. 2*

*Fig. 3*

*Fig. 4*

*Fig. 1*